# EUROPEAN PATENT APPLICATION

(11) **EP 2 666 497 A1**
(43) Date of publication of application: **27.11.2013**
(21) Application number: 13169091.9
(22) Date of filing: 24.05.2013
(51) Int. Cl.: A61M 15/00

(54) **An inhaler comprising a mouthpiece having an improved air channel**

(30) Priority: 25.05.2012 TR 201206167
(71) Applicant: Arven Ilac Sanayi Ve Ticaret A.S., Istanbul 34460 (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The present invention relates to a dry powder inhaler device (4), comprising a blister-advancing mechanism having a plurality of cavities, an external mouthpiece (1), an internal mouthpiece (2), and a medicament flow channel (3), characterized by comprising at least one medicament-free air channel (5), which is formed independently or separately from said medicament flow channel to prevent the blocking of air flow by the mouth of the respective user, and to provide a medicament-free air inhalation by the user when said device is in use.

## Description

### Field of Invention

The present invention relates to a device for administering dry powder inhalation medicaments.

The present invention particularly relates to improvements made in the mouthpiece of dry powder inhaler devices.

### Prior Art

Diseases such as asthma, bronchitis, and COLD (Chronic Obstructive Lung Disease) substantially decrease the quality of human life, despite the developments which have been carried out in the diagnosis and therapy thereof in the recent years. It has been proposed to administer medicaments via inhalers for optimizing the treatment of such diseases. The inhaler route of treatment is the most preferred one and it is expected to remain so, as the first option, in the future. The most important advantage of using medicaments via inhalation is based on providing a more efficient therapy by making use of lesser medicaments, delivering higher concentrations of medicaments to the airways, and particularly decreasing the systemic side effects of medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

There have been developed various inhalation devices for administering inhalation medicaments nowadays. These devices are basically classified into two groups, i.e. metered dose inhalers and dry powder inhalers. This type of devices are structurally provided with basic components such as an actuator, counter, housing, mouthpiece, lid, lock, etc.. Additionally, powder inhalation medicaments are kept in reservoirs or containers such as blisters, capsules, etc. Blisters are structured from two basic parts, a main layer provided with cavities holding the medicament, and a strippable protective layer.

A user inhales the respective medicament by means of a mouthpiece provided at the respective dry powder inhaler device with the aid of his/her breath, so that the powder medicament is delivered to the lungs, the target organs. The medicament, which is released with the opening of a blister or with the explosion of a capsule in blister- or capsule-inhalers is guided to the mouthpiece and is kept in a site which is in connection with the mouthpiece. The mouthpiece, in turn, is designed with a volume and length to comply with the anatomy of human mouth. Thus, both the administration of medicament is facilitated and hygiene compliance is ensured. Inhaling air into the lungs is an active action. With the contraction of inspiration muscles, the front and rear diameters of the chest cage are expanded and is elongated from top to down. According to the law of Boyle-Mariotte, when the volume of a gas increases, its pressure drops down. According to that law, since the pressure of air in the lungs will be lower in expanding lungs than that of atmospheric air, atmospheric air will fill the lungs. Air flow resulting from atmospheric air inhaled into the lungs allows the administration of powder medicament without requiring any other force. The powder medicament in the device is delivered into the lungs together with air flow. In this process, the amount of inhaled air and the time during which the air is inhaled are quite important. If the mouthpiece has a structure which would not allow the required air flow, the amount of medicament to be taken via inhalation shall be lowered. In this case, more than one inhalation or intermittent inhalations will occur, and no efficient amount of medicament will be delivered to the target organ. Additionally, since inhalation (and exhalation) ability of those individuals to take the medicament is already problematic, it will be impossible to reach the efficient amount of the respective medicament. In fact, an air aperture is necessary next to the medicament channel in mouthpieces for providing an easy inhalation of atmospheric air in mouthpieces.

Some of the currently-available mouthpiece embodiments are as follows.

The application EP0481666 discloses an inhaler device, which is activated upon inhalation by a user and has a plurality of medicament-containing compartments. A mouthpiece and an air inlet provided in each compartment ensure the activation of the device with a negative vacuum effect generated by the user of the device. This type of devices, however, are disadvantageous in terms to carrying, hygiene, and use of the device. For instance, the respective medicament cannot be delivered completely and no complete hygiene can be ensured.

The application EP0211595, in turn, discloses a device which provides for the administration of a medicament with the inhalation of a user, as well as an air channel which is opened to the exterior in the mouthpiece of the device. Since this aperture is made between the outer wall and inner wall of the mouthpiece, it may be closed by the lips of the user during use, or the user cannot use the mouthpiece at a desired efficiency anatomically so that the air flow regulation becomes difficult. Additionally, the medicament can escape through this aperture during air flow, or a risk may occur in which foreign substances may enter from the exterior. The fact that these apertures are made larger or smaller than required makes it difficult to reach desired levels of air flow pressure and prevent an ideal administration of the medicament.

In result, there is a novelty required in the field of inhaler devices, providing high-accuracy operation, as well as advantages in terms of hygiene and use.

### Objects and Brief Description of Invention

The present invention relates to an inhaler device having an improved mouthpiece for use in inhaling dry powders, eliminating all aforesaid problems and bringing additional advantages to the relevant prior art.

Accordingly, the main object of the present invention is to provide an inhaler device comprising a mouthpiece having an air channel which is situated so that the air flow is not blocked by the respective user during use.

Another object of the present invention is to provide an inhaler device comprising a mouthpiece which can be used at a desired accuracy and has optimum air flow limits.

A further object of the present invention is to provide an inhaler device comprising a mouthpiece which provides hygiene and easy of use.

Another object of the present invention is to provide an inhaler device comprising a mouthpiece, which allows a user to inhale and receive a medication at an user-dependent rate, rhythm, and time, without encountering any interruptions during inhalation.

In order to achieve all objects referred to above and to emerge from the following detailed description, a novelty is made in a dry powder inhaler device, which comprises a body including a blister-advancing mechanism with a plurality of cavities, a medicament flow channel, of which one end is in direct communication with at least one of said blister cavities, and the other end passes through an internal mouthpiece, which is in the form of an extension of said body, and opens to the exterior of the body and terminates there, and a mountable/dismountable external mouthpiece enveloping this internal mouthpiece.

In a preferred embodiment of the present invention, at least one medicament-free air channel is embodiment, which is formed independently, or separately, from said medicament flow channel to prevent the blocking of air flow by the mouth of the respective user, and to provide a medicament-free air inhalation by the user when said device is in use.

In a preferred embodiment of the present invention, said air channel is situated under said external mouthpiece.

In a preferred embodiment of the present invention, said air channel is formed in between the external mouthpiece and the internal mouthpiece.

In a preferred embodiment of the present invention, at least some part of the lateral surface of the internal mouthpiece is cut off, or removed, in order to form the air channel between the external mouthpiece and the internal mouthpiece.

In another preferred embodiment of the present invention, said air channel is terminated by a container which is opened to the interior of the body.

In a preferred embodiment of the present invention, said air channel enables an air flow rate between 20 - 50 Umin.

In another preferred embodiment of the present invention, the proportion of the flow rate of air through the air channel (5) to the flow rate of air through the medicament flow channel (3) is between 0.4 - 2.0.

In a preferred embodiment of the present invention, the number of said air channels is preferably two.

Structural and characteristic features, and all advantages of the present invention shall be made clear by means of annexed figures described here below and a detailed description written by making references to said figures; therefore, the present invention must be evaluated by taking into consideration these figures and the detailed description as well.

### Brief Description of Figures

Figure 1 is an illustration of a representative embodiment of the inhaler device according to the present invention.
Figure 2 is an demounted illustration of a representative embodiment of the inhaler device according to the present invention.
Figure 3 is an internal illustration of a representative embodiment of a part of the body of said device.
Figure 4 is a top illustration of a representative embodiment of the device according to the present invention.
Figure 5 is a side illustration of a representative embodiment of the device according to the present invention.

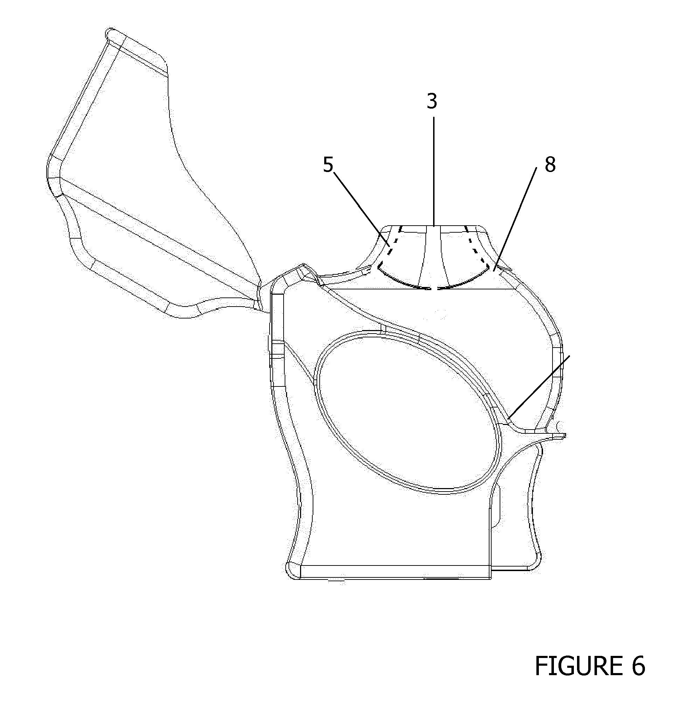

Figure 6 is a top illustration of a representative embodiment of the device according to the present invention.

### Reference Numbers in Figures

- 1.: external mouthpiece
- 2.: internal mouthpiece
- 2.1: partial surface of internal mouthpiece
- 3.: medicament flow channel
- 4.: inhaler device
- 5.: air channel
- 6.: body
- 7.: interior of body
- 8.: container

### Detailed Description of Invention

In the following detailed description, the inhaler device (4) according to the present invention shall be described illustratively by making references to annexed figures, only to make it clear without imposing any restrictions thereon.

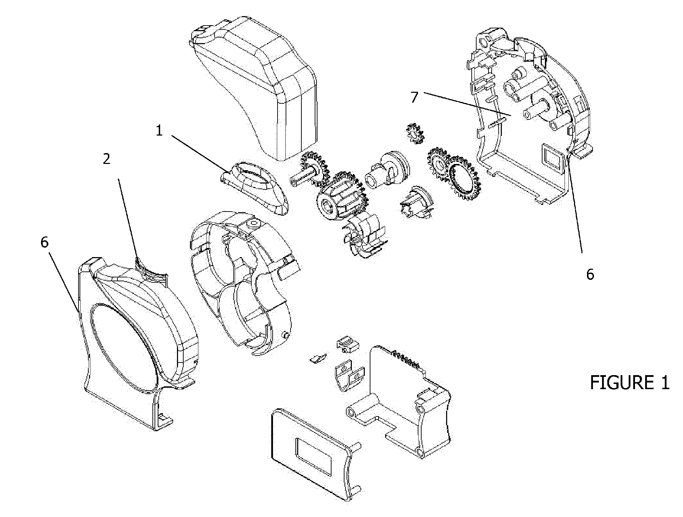

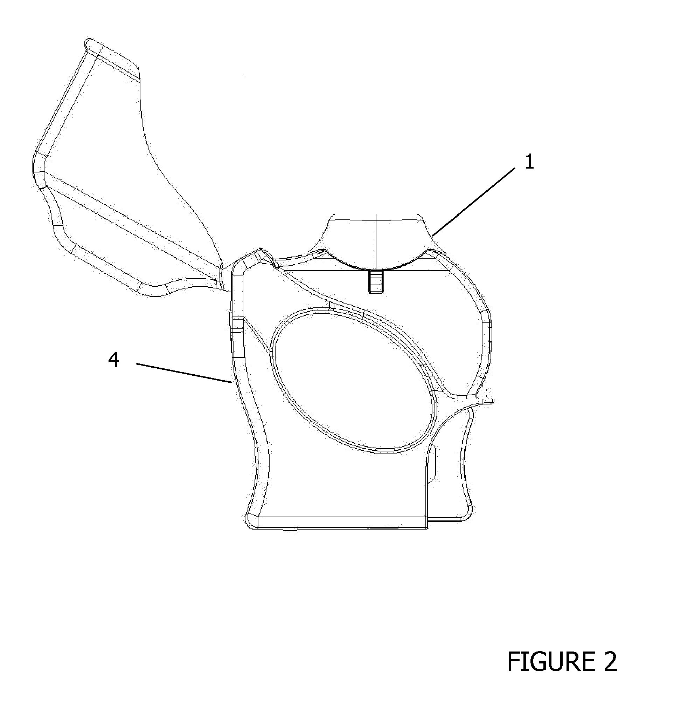

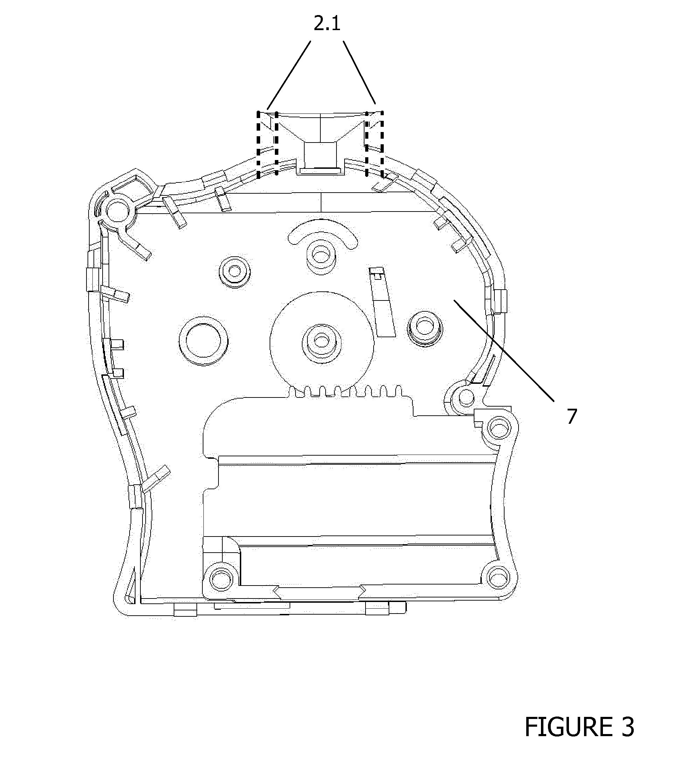

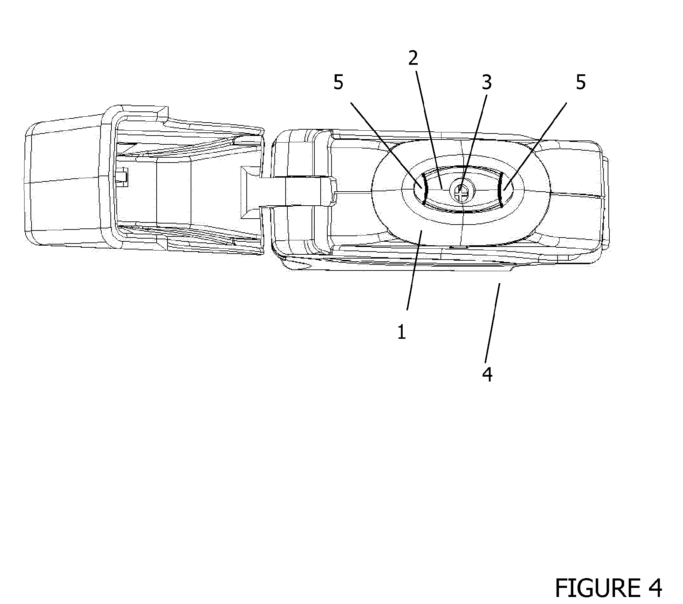

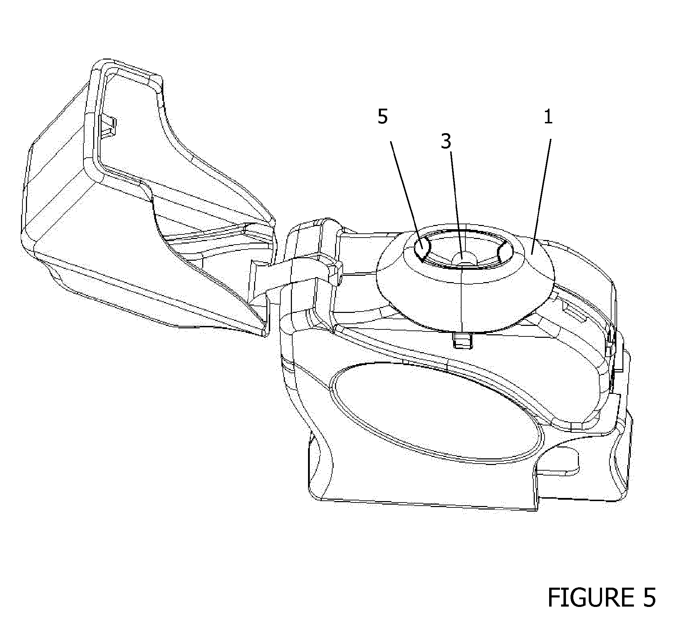

The present invention of which representative embodiments are shown in figures 1, 2, 3, 4, and 5, has a body (6) comprising a blister-advancing mechanism with a plurality of cavities. An internal mouthpiece (2), which is formed as an extension of the body is provided just above the latter. A removable external mouthpiece (1) is provided as well, which envelopes said internal mouthpiece and is closed thereon. Additionally, a medicament flow channel (3) is provided, of which one end is in direct communication with at least one of said blister cavities, and the other end passes through the internal mouthpiece (2), which is an extension of said body (6), and opens to the exterior of the body and terminates there. At least one air channel (5) is also provided, which is situated under said external mouthpiece (1) and is formed independently from said medicament flow channel to provide a medicament-free air flow. In fact, some part of the lateral surface component (2.1) of the internal mouthpiece is removed to form an air channel between the external mouthpiece (1) and the internal mouthpiece (2). Said air channel is formed by means of a cavity which is formed by a surface component removed between the external mouthpiece (1) and internal mouthpiece (2). Said cavity forms the air channel (5) and is terminated by a container (8) which is opened to the body's interior (7). Air circulation within the interior of body, in turn, is ensured by means of air hole(s) formed on the body. The number of air channels (5) is preferably two. Both sides of the interior mouthpiece is cut to some extent and narrowed from both sides, so that a structure is provided which enables an air flow at a desired efficiency and does not have any contact or communication with the medicament flow channel. During use, the contraction of inspiration muscles of the respective user results in an expansion of the front and rear diameters of his/her chest cage and in an elongation from top to down. Since the pressure of air in expanding lungs will be lower than that of the atmospheric air, atmospheric air will fill the lungs. Air flow resulting from atmospheric air inhaled into the body through the air channel (5) enables the administration of powder medicament without requiring any other force. The medicament is delivered into the body by means of a medicament flow channel (3), of which one end is in a blister cavity which is peeled off by means of the respective mechanism, and the other end is in an extension of said body (6) and passes through the internal mouthpiece (2) and opens to the exterior of the body and terminates there. The powder medicament in the device is delivered into the body via the air flow.

The present invention comprises a body (6) including a blister-advancing mechanism with a plurality of cavities, a medicament flow channel (3), of which one end is in direct communication with at least one of said blister cavities, and the other end passes through an internal mouthpiece (2), which is in the form of an extension of said body (6), and opens to the exterior of the body and terminates there, and a mountable/dismountable external mouthpiece (1) enveloping this internal mouthpiece, characterized by comprising at least one air channel (5), which is provided under said external mouthpiece (1) to prevent the blocking of air flow by the mouth of the respective user, and which is formed independently from said medicament flow channel to provide a medicament-free air inhalation by the user when said device is in use.

Air flow cannot be blocked by the user and the mouth of the user due to the position of the air channel during use. The device operates at a desired accuracy and has optimum air flow limits. Hygiene and easy of use are provided with the present invention.

Additionally, the respective user is enabled to use said device at an user-dependent rate, rhythm, and time, without facing any interruptions during use or inhalation. The cases are avoided, in which complete dose delivery fails during inhalation due to physical incapability and/or other inabilities resulting from the feeling of labored breathing, which leads to interruptions in inhalation.

Additionally, said air channel enables an air flow rate between 20 - 50 L/min. The proportion of the flow rate of air through the air channel (5) to the flow rate of air through the medicament flow channel (3) is between 0.4 - 2.0. Thanks to this embodiment, the efficient amount of a medicament is delivered completely, without requiring the respective user to exert an excessive force for a single inhalation. When currently-available inhaler devices are used, an air flow is provided which corresponds to a flow are of around 40-45 Umin if it is inhaled through the medicament flow channel only, and this leads to a feeling of inadequacy in terms of the inhaled medicament on the user side. As a result of this, the respective user terminates the use so that the medicament cannot be taken in full dose. The medicament flow channel cannot be expanded, since it will cause an incompliance in terms of the medicament blister. Since the powder in a blister cavity is to pass through a certain air channel to be delivered to the user, enlarging the medicament outlet channel will not give the desired effect, as the air inlet site of said medicament flow channel will be limited by the size of the cavity. This is because the cavity size has a determining effect on the amount of air passing through the medicament flow channel. Therefore, an air channel is provided here, which is separate from the medicament flow channel, so that the size of the apertures of the air inlet and air outlet can be adjusted as desired. When the amount of air cannot reach the pressure force to provide the transfer of medicament, the desired medicament amount cannot be delivered. Accordingly, an air channel is provided here, which is separate from the medicament flow channel, to eliminate all these problems. Thus, users are prevented from closing the air channel, either intentionally or unintentionally, during use. The air channel, enabling an air flow rate between 20 - 50 Umin, provides for a complete and smooth inhalation. Thanks to the separate air channel, the air flow rate is increased from around 43 Umin to 72 L/min.

In consequence, an extremely-accurate and safely-operable inhaler device is obtained with the embodiment disclosed above.

Said medicament is selected from the group consisting of at least one or a mixture of ciclesonide, budesonide, fluticasone, aldosterone, beklometazone, betametazone, chloprednol, cortisone, cortivasole, deoxycortone, desonide, desoxymetasone, dexametasone, difluorocortolone, fluchlorolone, flumetasone, flunisolide, fluquinolone, fluquinonide, flurocortisone, fluorocortolone, flurometolone, flurandrenolone, halcynonide, hydrocortisone, icometasone, meprednisone, methylprednisolone, mometasone, paramethasone, prednisolone, prednisone, tixocortole, triamcynolondane, salmeterol, ormoterol, arformoterol, salbutamol, indacaterol, terbutaline, metaproterenol, vilanterol, carmoterol, olodaterol, bambuterol, clenbuterol, tiotropium, glycopyronium, aclidinium, darotropium and ipratropium.

The air flow rates of the subject device are as follows. The following data were measured with a 4 kPa pressure drop according to European Pharmacopoeia 7.0 with an amount of inhalation of 4 L.

| | Air flow rate of medicament flow channel (L/min) | Air flow rate of air flow channel (L/min) | Total |
|---|---|---|---|
| | 41.5 | 29.1 | 70.6 |
| | 43.8 | 28.3 | 72.1 |
| | 44.7 | 25.4 | 70.1 |
| | 42.8 | 27.2 | 70 |
| | 44.4 | 25.8 | 70.2 |
| | 40.3 | 34.2 | 74.5 |
| | 42.8 | 26.0 | 68.8 |
| | 42.3 | 27.7 | 70 |
| | 45.7 | 29.7 | 75.4 |
| | 43 | 30.2 | 73.2 |
| mean | 43.1 | 28.4 | 71.5 |
| SD | 1.6 | | 2.2 |
| RSD (%) | 3.7 | | 3.1 |

The design of components used may be varied in alternative embodiments according to the type of device being produced. In result, the protection scope of the present invention is set forth in appended claims and cannot be restricted to the illustrative disclosures given above, under the detailed description. It is obvious that a person skilled in the relevant art can produce similar embodiments under the light of the foregoing disclosures, without departing from the main principles of the present invention.

## Claims

1. A dry powder inhaler device (4) comprising a blister-advancing mechanism having a plurality of cavities, an external mouthpiece (1), an internal mouthpiece (2), and a medicament flow channel (3); **characterized by** comprising at least one medicament-free air channel (5), which is formed independently or separately from said medicament flow channel to prevent the blocking of air flow by the mouth of the respective user, and to provide a medicament-free air inhalation by the user when said device is in use.

2. A dry powder inhaler device (4) according to Claim 1, wherein said air channel (5) is situated under said external mouthpiece (1).

3. A dry powder inhaler device (4) according to any of the preceding claims, wherein said device comprises a body (6).

4. A dry powder inhaler device (4) according to any of the preceding claims, comprising a body (6) including a blister-advancing mechanism with a plurality of cavities, a medicament flow channel (3), of which one end is in direct communication with at least one of said blister cavities, and the other end passes through an internal mouthpiece (2), which is in the form of an extension of said body (6), and opens to the exterior of the body and terminates there, and a mountable/dismountable external mouthpiece (1) enveloping this internal mouthpiece; **characterized by** comprising at least one air channel (5), which is provided under said external mouthpiece (1) to prevent the blocking of air flow by the mouth of the respective user, and which is formed separately from said medicament flow channel to provide a medicament-free air inhalation by the user when said device is in use.

5. A dry powder inhaler device (4) according to any of the preceding claims, wherein said air channel is formed in between the external mouthpiece (1) and internal mouthpiece (2).

6. A dry powder inhaler device (4) according to any of the preceding claims, **characterized in that** at least some of the lateral surface part (2.1) of the internal mouthpiece is cut off, or removed, in order to form the air channel between the external mouthpiece (1) and the internal mouthpiece (2).

7. A dry powder inhaler device (4) according to any of the preceding claims, wherein said air channel (5) is terminated by a container (8) which opens to the interior (7) of said body.

8. A dry powder inhaler device (4) according to any of the preceding claims, wherein said air channel (5) enables an air flow rate of between 20 - 50 Umin.

9. A dry powder inhaler device (4) according to any of the preceding claims, wherein the proportion of the flow rate of air through the air channel (5) to the flow rate of air through the medicament flow channel (3) is between 0.4 - 2.0.

10. A dry powder inhaler device (4) according to any of the preceding claims, wherein the number of said air channels (5) is preferably two.
